# EUROPEAN PATENT APPLICATION

(11) **EP 2 927 226 A1**
(43) Date of publication of application: **07.10.2015**
(21) Application number: 13845572.0
(22) Date of filing: 11.10.2013
(51) Int. Cl.: C07D 403/10, A61K 31/506, A61P 9/12

(54) **MONOHYDRATE CRYSTAL OF FIMASARTAN POTASSIUM SALT, METHOD FOR PREPARING SAME, AND PHARMACOLOGICAL COMPOSITION COMPRISING SAME**

(30) Priority: 12.10.2012 KR 20120113848
(71) Applicant: Boryung Pharmaceutical Co., Ltd., Seoul 110-750 (KR)
(72) Inventor: KIM, Je Hak, Anyang-si Gyeonggi-do 431-050 (KR); KIM, Ji Han, Seoul 150-823 (KR); LEE, Joon Kwang, Gwangmyeong-si Gyeonggi-do 423-774 (KR); YOO, Byoung Wug, Anyang-si Gyeonggi-do 431-708 (KR); HAN, Nam Seok, Ansan-si Gyeonggi-do 426-811 (KR); NAM, Kyung Wan, Gunpo-si Gyeonggi-do 435-040 (KR); KIM, Chang Mo, Gunpo-si Gyeonggi-do 435-050 (KR); LEE, Joo Han, Seoul 121-010 (KR)
(74) Representative: D Young & Co LLP
(86) International application number: PCT/KR2013/009097
(87) International publication number: WO 2014/058268

(57) **Abstract**

The present invention relates to a novel monohydrate crystal form of fimasartan potassium salt, which is excellent in moisture and temperature stability, and which has a crystal particle size easy to prepare the formulation and superior homogeneity. The present invention provides a novel fimasartan potassium salt monohydrate.

## Description

### [Technical Field]

The present invention relates to a monohydrate crystal form of fimasartan potassium salt, a method for preparing the same, and a pharmaceutical composition comprising the same and, more particularly, the present invention relates to a monohydrate crystal form of fimasartan potassium salt, a method for preparing the same and a pharmaceutical composition including the same, which has antagonism to an angiotensin II receptor and is a form in which a fimasartan potassium salt compound useful for the treatment of cardiovascular diseases due to the angiotensin II receptor can be stably present in the air.

### [Background Art]

Fimasartan, an angiotensin II receptor antagonist, has been approved for the treatment of hypertension and is commercially available in two doses of 60 mg and 120 mg under the trade name of Kanarb, and in particular its active ingredient is a fimasartan potassium salt trihydrate.

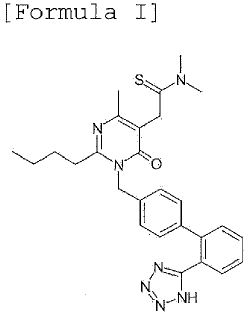

Fimasartan is a pyrimidinone compound, which is different from existing drugs having antagonism to angiotensin II receptors and is disclosed in International Patent Application No. PCT/KR1999/00198, and a novel method for preparing fimasartan is disclosed in Korean Patent Publication No. 10-2001-0090193.

Moreover, Korean Patent Publication No. 10-2004-0032639 discloses the preparation of a trihydrate of fimasartan potassium salt.

As disclosed in Example 5 and Table 1 of Korean Patent Publication No. 10-2004-0032639, the trihydrate of fimasartan potassium salt is converted into the form of an anhydride when dried at 60 °C for 24 hours, which is slowly converted to a trihydrate when left at room temperature and may be more quickly converted to the trihydrate under the condition having high humidity above predetermined value.

Furthermore, depending on the drying temperature, the loss of crystallization water occurs in the fimasartan potassium salt trihydrate, resulting in changes in water content, and an additional process is required in order to prepare a fimasartan potassium salt trihydrate that is more stable at room temperature.

For example, after the preparation of the fimasartan potassium salt trihydrate, the water content may vary depending on the temperature conditions in the drying process and may be easily changed depending on the drying conditions during the preparation of the formulation.

These problems require a more detailed and complicated process in dealing with the fimasartan potassium salt trihydrate, and in order to make up for these problems, it is necessary to maintain a constant humidity so as to prevent the loss of the crystallization water in the trihydrate or to add a process for supplementing the lost crystallization water to convert into a more stable fimasartan potassium salt trihydrate, which is very cumbersome. Moreover, the increase in processing costs due to the addition of the process leads to economic loss, resulting in many difficulties.

Accordingly, the present inventors have studied to develop a novel crystalline form of fimasartan potassium salt, which has excellent property in moisture and temperature stability and a uniform particle size that does not require a separate milling process, and completed the present invention.

### Prior Art Document

Patent Document:
Korean Patent Publication No.: 10-2004-0032639

### [Disclosure]

### [Technical Problem]

Accordingly, the present invention provides a novel crystalline form of fimasartan potassium salt monohydrate that is excellent in moisture and temperature stability and has a uniform particle size.

Moreover, the present invention provides a method for preparing the novel crystalline form of fimasartan potassium salt monohydrate.

Furthermore, the present invention provides a pharmaceutical composition for the prevention, alleviation, or treatment of hypertension, comprising the novel crystalline form of fimasartan potassium salt monohydrate.

### [Technical Solution]

The present invention relates to a novel crystalline form of fimasartan potassium salt monohydrate, which is excellent in moisture and temperature stability and has a particle size that is uniform and easy to prepare a formulation, and provides a fimasartan potassium salt monohydrate.

Compared to the commercially available fimasartan potassium salt trihydrate used in Kanarb, the fimasartan potassium salt monohydrate according to the present invention has little or no change in water content due to the temperature and thus is highly stable to the temperature change. Moreover, the fimasartan potassium salt monohydrate according to the present invention has a uniform particle size which is easy to prepare the formulation and thus the processing time for preparing the formulation is significantly reduced.

The present invention provides a novel fimasartan potassium salt monohydrate.

The crystalline form of the fimasartan potassium salt monohydrate of the present invention has X-ray powder diffraction spectrum peaks using Cu-Kα radiation in diffraction angles 2θ value shown at 6.67±0.2, 7.62±0.2, 11.03+0.2, 15.32+0.2, 16.49+0.2, 20.12±0.2, 25.65±0.2, and 27.28±0.2.

Preferably, the crystalline form of fimasartan potassium salt monohydrate of the present invention has X-ray powder diffraction spectrum peaks using Cu-Kα radiation in diffraction angles 2θ value shown at 6.67+0.1, 7.62+0.1, 11.03±0.1, 15.32±0.1, 16.49±0.1, 20.12+0.1, 25.65±0.1, and 27.28±0.1.

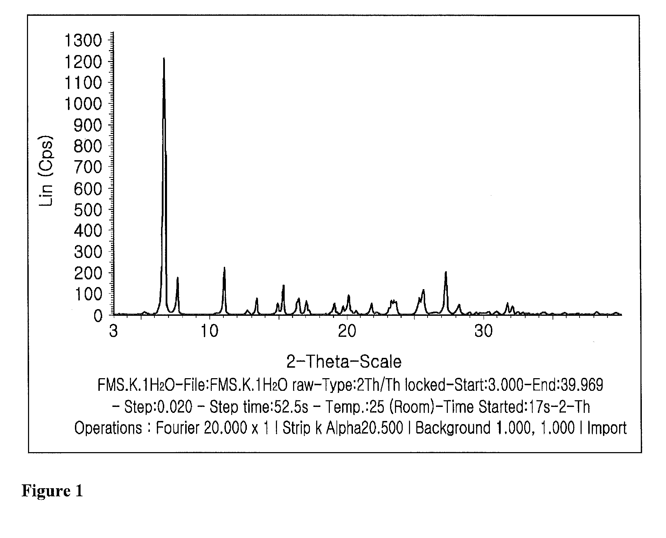

More preferably, the crystalline form of fimasartan potassium salt monohydrate of the present invention has X-ray powder diffraction spectrum peaks shown in FIG. 1.

The crystalline form of fimasartan potassium salt monohydrate of the present invention has an endothermic transition temperature in differential scanning calorimetry (DSC) at about 267 °C to about 268 °C when the heating rate is 10 °C/min.

However, the DSC endothermic transition temperature may vary depending on the purity of the crystalline form of the present invention and may have a value in the range of about 263 °C to about 269 °C, for example. Moreover, this value may vary depending on the heating rate of a device for measuring the DSC endothermic transition temperature.

Moreover, the crystalline form of fimasartan potassium salt monohydrate of the present invention exhibits a change in water content of about 3.4% measured using a thermogravimetric/differential thermal analyzer.

Accordingly, the present invention provides a novel crystalline form of fimasartan potassium salt monohydrate that is excellent in moisture and temperature stability and has a uniform particle size.

The present invention provides a method for preparing a fimasartan potassium salt monohydrate. The method includes crystallizing fimasartan using an organic solvent including at least one selected from the group consisting of methanol, ethanol, isopropanol, ethyl acetate, tetrahydrofuran, acetonitrile, acetone, and dioxane.

The method for preparing the fimasartan potassium salt monohydrate may include:
a first step of preparing a suspension by adding the fimasartan to an organic solvent including at least one selected from the group consisting of methanol, ethanol, isopropanol, ethyl acetate, tetrahydrofuran, acetonitrile, acetone, and dioxane;
a second step of preparing a reactant by adding a mixed solution including potassium hydroxide and 2-ethylhexanoic acid to the suspension;
a third step of obtaining a crystal by cooling the reactant; and
a fourth step of drying the crystal.

The fimasartan, a starting material, and the reagents used in the preparation method of the present invention can be commercially available.

In the first step of the preparation method of the present invention, the suspension may be preferably prepared in an amount of 2 mL to 5 mL with respect to 1 g of fimasartan, more preferably in an amount of 2 mL to 3 mL.

In the first step of the preparation method of the present invention, the preparation of the suspension may be preferably performed at 20 °C to 30 °C, more preferably at 25 °C.

In the present invention, the organic solvent used in the preparation of the suspension may be isopropyl alcohol.

In the second step of the preparation method of the present invention, the mixed solution may further comprise isopropyl alcohol.

In the second step of the preparation method of the present invention, the step of preparing the reactant may be performed by adding the mixed solution to the suspension, followed by heating and stirring under reflux.

The heating of the reactant may be preferably performed at 70 °C to 90 °C, more preferably 78 °C to 84 °C. The fimasartan can be dissolved by the heating.

In the third step of the preparation method of the present invention, the cooling may be preferably performed at 20 °C to 30 °C, more preferably at 25 °C.

The stirring under reflux may be preferably performed for 30 minutes to 2 hours in order to obtain a product in high yield and high purity.

In the fourth step of the preparation method of the present invention, the drying may be performed by drying under reduced pressure.

In the fourth step of the preparation method of the present invention, the drying may be preferably performed for 5 hours to 10 hours, more preferably for 8 hours.

In the fourth step of the preparation method of the present invention, the drying may be preferably performed at 35 °C to 45 °C, more preferably at about 40 °C.

In the fourth step of the preparation method of the present invention, the drying may be preferably performed at 20 mmHg to 5 mmHg.

The present invention provides a method for preparing a fimasartan potassium salt monohydrate. The method includes crystallizing a fimasartan potassium salt trihydrate using an organic solvent comprising at least one selected from the group consisting of methanol, ethanol, isopropanol, ethyl acetate, tetrahydrofuran, acetonitrile, acetone, and dioxane.

The method for preparing fimasartan potassium salt monohydrate may include:
a first step of preparing a reactant by adding the fimasartan potassium salt trihydrate to an organic solvent comprising at least one selected from the group consisting of methanol, ethanol, isopropanol, ethyl acetate, tetrahydrofuran, acetonitrile, acetone, and dioxane, followed by heating and stirring;
a second step of obtaining a crystal by cooling the reactant; and
a third step of drying the crystal under reduced pressure.

The fimasartan potassium salt trihydrate, a starting material and the reagents used in the preparation method of the present invention can be commercially available.

In the first step of the preparation method of the present invention, the heating may be performed at 70 °C to 80 °C. The fimasartan trihydrate can be dissolved by the heating.

In the first step of the preparation method of the present invention, the organic solvent may be ethanol. Otherwise, the organic solvent may be a mixed solvent including at least two solvents selected from the group consisting of methanol, acetonitrile, tetrahydrofuran, ethyl acetate, acetone, and dioxane.

In the first step of the preparation method of the present invention, the stirring may be preferably performed for more than 10 hours, more preferably for 10 to 20 hours, most preferably for 15 hours.

In the second step of the preparation method of the present invention, the cooling may be preferably performed at 20 °C to 30 °C, more preferably at 25 °C.

In the third step of the preparation method of the present invention, the drying may be preferably performed for 5 hours to 10 hours, more preferably for 8 hours.

In the third step of the preparation method of the present invention, the drying may be preferably performed at 35 °C to 45 °C, more preferably at about 40 °C.

In the third step of the preparation method of the present invention, the drying may preferably be performed at 20 mmHg to 5 mmHg.

Moreover, the method for preparing a fimasartan potassium salt monohydrate may includes:
preparing a reactant by adding the fimasartan potassium salt trihydrate to an organic solvent comprising at least one selected from the group consisting of methanol, ethanol, isopropanol, ethyl acetate, tetrahydrofuran, acetonitrile, acetone, and dioxane, followed by reflux;
obtaining a crystal by cooling and stirring the reactant; and
drying the crystal under reduced pressure.

The fimasartan potassium salt trihydrate, a starting material, and the reagents used in the preparation method of the present invention can be commercially available.

In the first step of the preparation method of the present invention, the organic solvent may include at least one selected from the group consisting of acetonitrile, tetrahydrofuran, ethyl acetate, acetone, and dioxane.

In the first step of the preparation method of the present invention, the reflux may be preferably performed for 10 minutes to 1 hour, more preferably for 30 minutes.

In the first step of the preparation method of the present invention, the stirring may be preferably performed for more than 10 hours, more preferably for 15 hours.

In the second step of the preparation method of the present invention, the cooling may be preferably performed at 20 °C to 30 °C, more preferably at 25 °C.

In the third step of the preparation method of the present invention, the drying may be preferably performed for 5 hours to 10 hours, more preferably for 8 hours.

In the third step of the preparation method of the present invention, the drying may be preferably performed at 35 °C to 45 °C, more preferably at about 40 °C.

In the third step of the preparation method of the present invention, the drying may preferably be performed at 20 mmHg to 5 mmHg.

Moreover, the present invention provides a fimasartan potassium salt monohydrate prepared by one of the above-described methods for preparing the fimasartan potassium salt monohydrate. However, the fimasartan potassium salt monohydrate of the present invention is not limited to these preparation methods.

The present invention provides a pharmaceutical composition for prevention, alleviation, or treatment of hypertension, including a fimasartan potassium salt monohydrate and a pharmaceutically acceptable carrier thereof.

As used herein, the term "pharmaceutically acceptable" means that the resulting composition is physiologically tolerable and generally does not induce allergic reactions such as gastroenteric trouble and dizziness or other similar reactions upon administration to an animal, preferably human. The pharmaceutically effective amount may vary appropriately depending on a disease and its severity, a patient's age, weight, health, and sex, administration route, treatment period, etc.

Examples of the pharmaceutically acceptable carrier may include at least one selected from the group consisting of lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate and mineral oil, propyl hydroxybenzoate, talc, magnesium stearate and mineral oil, dextrin, calcium carbonate, propylene glycol, liquid paraffin, and physiological saline, but not limited thereto, and further include typical carriers, excipients, or diluents.

Moreover, the pharmaceutical composition for prevention, alleviation, or treatment of hypertension may further include fillers, extenders, binders, disintegrant, anticoagulants, lubricants, wetting agent, pH adjustor, nutrients, vitamins, electrolytes, alginic acids and salt thereof, pectic acids and salt thereof, protective colloids, glycerin, flavourings, emulsifiers, preservatives, etc. The above-mentioned ingredients may be added alone or in combination to the pharmaceutical composition for prevention, alleviation, or treatment of hypertension or the fimasartan potassium salt monohydrate.

The pharmaceutical composition of the present invention may be administered via any administration route as long as it can reach a target tissue.

The composition of the present invention may be administered orally, intraperitoneally, intravenously, intramuscularly, subcutaneously, intradermally, intranasally, intrapulmonary, rectally, or subdurally, but not limited thereto, and may be preferably administered orally.

The dose of the pharmaceutical composition according to the present invention varies depending on a patient's weight, age, sex, health, diet, administration time, administration route, excretion rate, and disease severity. The daily dose of an active ingredient of the pharmaceutical composition is in the range of 0.1 to 1000 mg, preferably 50 to 500 mg, and may be administered once or several times a day.

The fimasartan potassium salt monohydrate according to the present invention may be contained in an amount of 20 to 80 wt% with respect to the total weight of the composition of the present invention, and when the composition of the present invention is prepared into a tablet, the monohydrate may be contained as a fimasartan potassium salt in an amount of more than 30 mg per tablet, preferably in an amount of 30, 60, 120, or 240 mg.

Moreover, the present invention provides a formulation including a fimasartan potassium salt monohydrate.

The formulation may include oral formulations such as granules, powders, liquids, tablets, capsules, or dry syrups, etc. or parenteral formulations such as injections, etc., but not limited thereto. Preferably, the formulation of the present invention may be in the form of a tablet or a capsule or may be in the form of a liquid or an injection.

### [Advantageous Effects]

The present invention provides a novel crystalline form of fimasartan potassium salt monohydrate that is excellent in moisture and temperature stability and has a uniform particle size that is easy to prepare a formulation.

### [Description of Drawings]

FIG. 1 shows the XRD diffraction pattern of a crystalline form of fimasartan potassium salt monohydrate according to the present invention.
FIG. 2 shows the DSC curve of a crystalline form of fimasartan potassium salt monohydrate according to the present invention.
FIG. 3 shows the XRD diffraction pattern of a crystalline form of fimasartan potassium salt trihydrate.
FIG. 4 shows the DSC curve of a crystalline form of fimasartan potassium salt trihydrate.

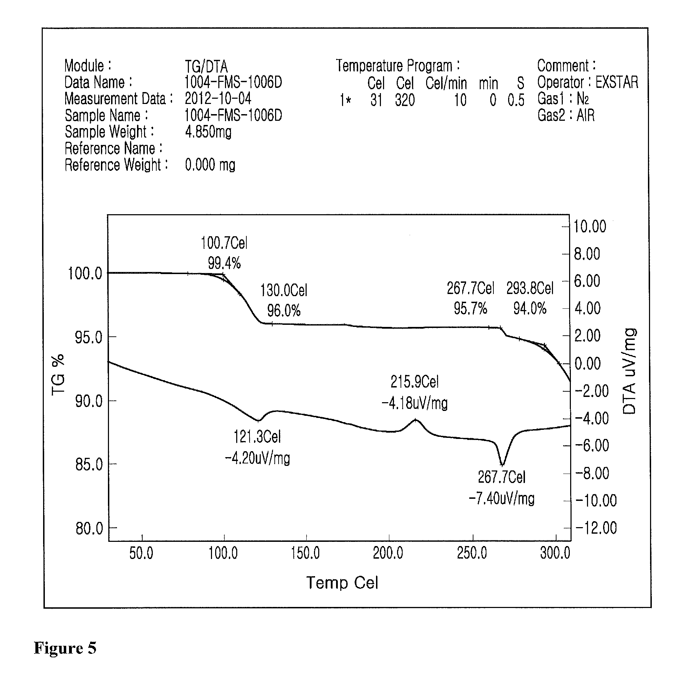

FIG. 5 shows the TG/DTA curves of a crystalline form of fimasartan potassium salt monohydrate according to the present invention.

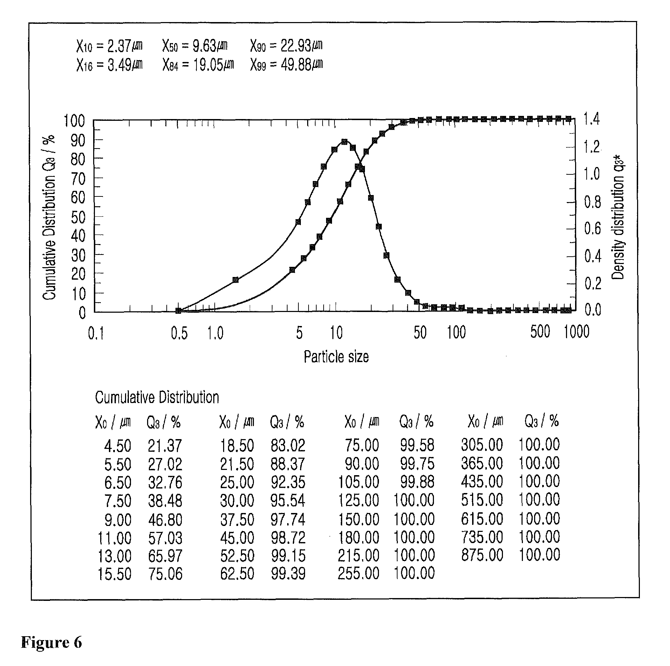

FIG. 6 shows the particle size of a crystalline form of fimasartan potassium salt monohydrate according to the present invention.

### [Mode for Invention]

Hereinafter, the present invention will be described in more detail with reference to the following Examples. However, these Examples are provided only to illustrate the present invention, but the scope of the present invention is not limited thereto.

Details of the reagents and solvents used in the present invention are as follows:
Fimasartan potassium salt trihydrate (Boryung Pharm. Serial number: 2005); IPA (Daejung, Serial number: I0077MD); KOH (Daejung, Serial number: P0215KA); 2-ethylhexanoic acid (Samchun, Serial number: 082511); MeOH (Samchun, Serial number: 061512); THF (Daejung, Serial number: T0017MD1); EtOH (Daejung, Serial number: E1946MG); EA (Daejung, Serial number: E0036MII1); ACN (Daejung, Serial number: A0049MG1); and AC (Daejung, Serial number: A0033MJ).

Unless otherwise particularly specified, the drying under reduced pressure mentioned below was performed using a vacuum oven (OV-12 manufactured by JEIO TECH, Korea) and a vacuum pump (MD 4C NT manufactured by Vacuubrand, Germany).

The yield measurement was performed on the following machines under the following conditions, and the measurement of the water content was performed by Karl Fischer (KF) method in METTLER TOLEDO V20 Volumetric KF Titrator using Merck's reagent (reacted with 5 mg H₂O in 1 mL).

X-ray diffraction spectrometry and differential scanning calorimetry (DSC) are as follows:
Powder X-ray diffraction spectrometer and conditions
   - Manufacturer: Bruker (Germany)
   - Model: D8 Focus
   - Kβ remover: Ni filter
   - Voltage: 40 kV
   - Current: 40 mA
   - Scan range: 3~40 deg.
   - Scan rate: 0.3 sec/step
   - Increment: 0.02 deg.
   - Divergence slit width: 0.6 mm
   - Air scatter slit: 3 mm
   - Detector: LynxEye Detector (line detector)
Differential scanning calorimeter and conditions
   - Manufacturer: Mettler Toredo
   - Model: DSC 1 STARE system
   - heating rate: 10 °C/min

### Example 1: Preparation of fimasartan potassium salt monohydrate

A suspension was prepared by adding 2-n-butyl-5-dimethylaminothiocarbonylmethyl-6-methy-3-[[2'-(1H-tetrazol-5-yl)biphenyl-4-yl]methyl]-pyrimidin-4(3H)-one (hereinafter referred to as fimasartan) (6.55 g, 12.6 mmol) to isopropyl alcohol (18.5 mL).

A solution prepared by mixing isopropyl alcohol (19.6 mL), potassium hydroxide (1.18 g, 21.0 mmol) and 2-ethylhexanoic acid (2.83 g, 19.6 mmol) was added to the prepared suspension, dissolved at 81 °C, and then stirred under reflux for 1 hour. Then, the mixture was cooled to 25 °C to precipitate a crystal, and the obtained solid was filtered and washed sequentially with isopropyl alcohol (2.5 mL) and ethyl acetate (2.0 mL).

Then, the crystal was dried under reduced pressure of 10 mmHg at 40 °C for 8 hours to yield a fimasartan potassium salt monohydrate as a white powder solid.
Yield: 6.49 g (95.5%)
water content (KF method): 3.88%

### Example 2: Preparation of fimasartan potassium salt monohydrate

A suspension was prepared by adding fimasartan (8.52 g, 16.4 mmol) to isopropyl alcohol (18.5 mL).

A solution prepared by mixing isopropyl alcohol (19.6 mL), potassium hydroxide (1.18 g, 21.0 mmol), and 2-ethylhexanoic acid (2.83 g, 19.6 mmol) was added to the prepared suspension, dissolved at 81 °C, and then stirred under reflux for 1 hour. Then, the mixture was cooled to 25 °C to precipitate a crystal, and the obtained solid was filtered and washed with isopropyl alcohol (2.5 mL) and ethyl acetate (2.0 mL).

Then, the crystal was dried under reduced pressure of 10 mmHg at 40 °C for 8 hours to yield a fimasartan potassium salt monohydrate as a white powder solid.
Yield: 8.35 g (94.4%)
water content (KF method): 3.70%

### Example 3: Preparation of fimasartan potassium salt monohydrate

A suspension was prepared by adding fimasartan (8.91 g, 17.1 mmol) to isopropyl alcohol (18.5 mL).

A solution prepared by mixing isopropyl alcohol (19.6 mL), potassium hydroxide (1.18 g, 21.0 mmol), and 2-ethylhexanoic acid (2.83 g, 19.6 mmol) was added to the prepared suspension, dissolved at 81 °C, and then stirred under reflux for 1 hour. Then, the mixture was cooled to 25 °C to precipitate a crystal, and the obtained solid was filtered and washed with isopropyl alcohol (2.5 mL) and ethyl acetate (2.0 mL).

Then, the crystal was dried under reduced pressure of 10 mmHg at 40 °C for 8 hours to yield a fimasartan potassium salt monohydrate as a white powder solid.
Yield: 8.65 g (93.8%)
water content (KF method): 3.44%

### Example 4: Preparation of fimasartan potassium salt monohydrate

Fimasartan potassium salt trihydrate (2.50 g, 4.2 mmol) was added to ethanol (5 mL), dissolved by heating at 75 °C, cooled to 25 °C to precipitate a crystal, and then stirred for 15 hours. The obtained solid was filtered at 25 °C and dried under reduced pressure of 10 mmHg at 40 °C for 8 hours to yield a fimasartan potassium salt monohydrate as a white powder solid.
Yield: 1.97 g (83.3%)
water content (KF method): 3.20%

### Example 5: Preparation of fimasartan potassium salt monohydrate

Fimasartan potassium salt trihydrate (2.50 g, 4.2 mmol) was added to a mixed solvent of acetonitrile (8 mL) and methanol (1.0 mL), dissolved by heating at 75 °C, cooled to 25 °C to precipitate a crystal, and then stirred for 15 hours. The obtained solid was filtered at 25 °C and dried under reduced pressure of 10 mmHg at 40 °C for 8 hours to yield a fimasartan potassium salt monohydrate as a white powder solid.
Yield: 1.65g (71.4%)
water content (KF method): 3.32%

### Example 6: Preparation of fimasartan potassium salt monohydrate

Fimasartan potassium salt trihydrate (10.0 g, 16.8 mmol) was added to a mixed solvent of tetrahydrofuran (100 mL) and methanol (5 mL), dissolved by heating at 75 °C, cooled to 25 °C to precipitate a crystal, and then stirred for 15 hours. The obtained solid was filtered at 25 °C and dried under reduced pressure of 10 mmHg at 40 °C for 8 hours to yield a fimasartan potassium salt monohydrate as a white powder solid.
Yield: 8.24g (87.7%)
water content (KF method): 3.60%

### Example 7: Preparation of fimasartan potassium salt monohydrate

Fimasartan potassium salt trihydrate (10.0 g, 16.8 mmol) was added to a mixed solvent of ethyl acetate (100 mL) and methanol (10 mL), dissolved by heating at 75 °C, cooled to 25 °C to precipitate a crystal, and then stirred for 15 hours. The obtained solid was filtered at 25 °C and dried under reduced pressure of 10 mmHg at 40 °C for 8 hours to yield a fimasartan potassium salt monohydrate as a white powder solid.
Yield: 8.19 g (87.2%)
water content (KF method): 3.64%

### Example 8: Preparation of fimasartan potassium salt monohydrate

Fimasartan potassium salt trihydrate (10.0 g, 16.8 mmol) was added to a mixed solvent of acetone (100 mL) and methanol (7.5 mL), dissolved by heating at 75 °C, cooled to 25 °C to precipitate a crystal, and then stirred for 15 hours. The obtained solid was filtered at 25 °C and dried under reduced pressure of 10 mmHg at 40 °C for 8 hours to yield a fimasartan potassium salt monohydrate as a white powder solid.
Yield: 6.24 g (66.4%)
water content (KF method): 3.99%

### Example 9: Preparation of fimasartan potassium salt monohydrate

Fimasartan potassium salt trihydrate (10.0 g, 16.8 mmol) was added to a mixed solvent of acetonitrile (100 mL) and methanol (5 mL), dissolved by heating at 75 °C, cooled to 25 °C to precipitate a crystal, and then stirred for 15 hours. The obtained solid was filtered at 25 °C and dried under reduced pressure of 10 mmHg at 40 °C for 8 hours to yield a fimasartan potassium salt monohydrate as a white powder solid.
Yield: 6.19 g (65.9%)
water content (KF method): 3.73%

### Example 10: Preparation of fimasartan potassium salt monohydrate

Fimasartan potassium salt trihydrate (10.0 g, 16.8 mmol) was added to a mixed solvent of dioxane (100 mL) and methanol (5 mL), dissolved by heating at 75 °C, cooled to 25 °C to precipitate a crystal, and then stirred for 15 hours. The obtained solid was filtered at 25 °C and dried under reduced pressure of 10 mmHg at 40 °C for 8 hours to yield a fimasartan potassium salt monohydrate as a white powder solid.
Yield: 7.98 g (85.0%)
water content (KF method): 3.01%

### Example 11: Preparation of fimasartan potassium salt monohydrate

Fimasartan potassium salt trihydrate (10.0 g, 16.8 mmol) was added to tetrahydrofuran (100 mL), subjected to reflux for 30 minutes, cooled to 25 °C, and then stirred for 15 hours. The obtained solid was filtered at 25 °C and dried under reduced pressure of 10 mmHg at 40 °C for 8 hours to yield a fimasartan potassium salt monohydrate as a white powder solid.
Yield: 8.62 g (91.7%)
water content (KF method): 3.85%

### Example 12: Preparation of fimasartan potassium salt monohydrate

Fimasartan potassium salt trihydrate (10.0 g, 16.8 mmol) was added to ethyl acetate (100 mL), subjected to reflux for 30 minutes, cooled to 25 °C, and then stirred for 15 hours. The obtained solid was filtered at 25 °C and dried under reduced pressure of 10 mmHg at 40 °C for 8 hours to yield a fimasartan potassium salt monohydrate as a white powder solid.
Yield: 8.56 g (91.1%)
water content (KF method): 3.77%

### Example 13: Preparation of fimasartan potassium salt monohydrate

Fimasartan potassium salt trihydrate (10.0 g, 16.8 mmol) was added to acetone (100 mL), subjected to reflux for 30 minutes, cooled to 25 °C, and then stirred for 15 hours. The obtained solid was filtered at 25 °C and dried under reduced pressure of 10 mmHg at 40 °C for 8 hours to yield a fimasartan potassium salt monohydrate as a white powder solid.
Yield: 8.49 g (90.4%)
water content (KF method): 3.10%

### Example 14: Preparation of fimasartan potassium salt monohydrate

Fimasartan potassium salt trihydrate (10.0 g, 16.8 mmol) was added to acetonitrile (100 mL), subjected to reflux for 30 minutes, cooled to 25 °C, and then stirred for 15 hours. The obtained solid was filtered at 25 °C and dried under reduced pressure of 10 mmHg at 40 °C for 8 hours to yield a fimasartan potassium salt monohydrate as a white powder solid.
Yield: 7.78 g (82.8%)
water content (KF method): 3.95%

The identification of the obtained fimasartan potassium salt monohydrate was performed by X-ray powder diffraction and differential scanning calorimetry.

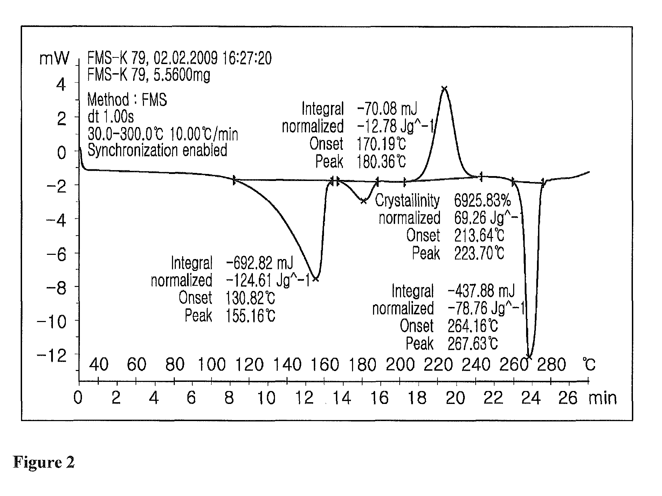

The XRD diffraction pattern of the obtained fimasartan potassium salt monohydrate by X-ray powder diffraction is shown in FIG. 1, and the DSC curve by differential scanning calorimetry is shown in FIG. 2, respectively.

### Comparative Example 1: Preparation of fimasartan potassium salt trihydrate

Fimasartan potassium salt trihydrate was prepared with reference to Example 5 of Korean Patent Publication No. 10-2004-0032639. A solution was prepared by dissolving 21.7 g of 2-n-butyl-5- dimethylaminothiocarbonylmethyl-6-methy-3-[[2'-(1H-tetrazol-5-yl)biphenyl-4-yl]methyl]-pyrimidin-4(3H)-one (fimasartan) in 55 mL of isopropanol. 60 mL of 1M KEH (2-ethylhexanoic acid + potassium hydroxide) isopropanol solution was added to the prepared solution, and the reactants were stirred under reflux for 4 hours and cooled to 25 °C, yielding a solid product. A compound obtained by filtering and drying the resulting solid product was added to a mixed solution of water and tetrahydrofuran (1:10), dissolved under reflux, and cooled to 25 °C to produce a crystal, and the obtained crystal was filtered and dried at 760 mmHg and 35 °C for 24 hours to yield a fimasartan potassium salt trihydrate.
Melting point: 265 °C (DSC)
water content (KF method): 9.15%

The identification of the obtained fimasartan potassium salt trihydrate was performed by X-ray powder diffraction and differential scanning calorimetry.

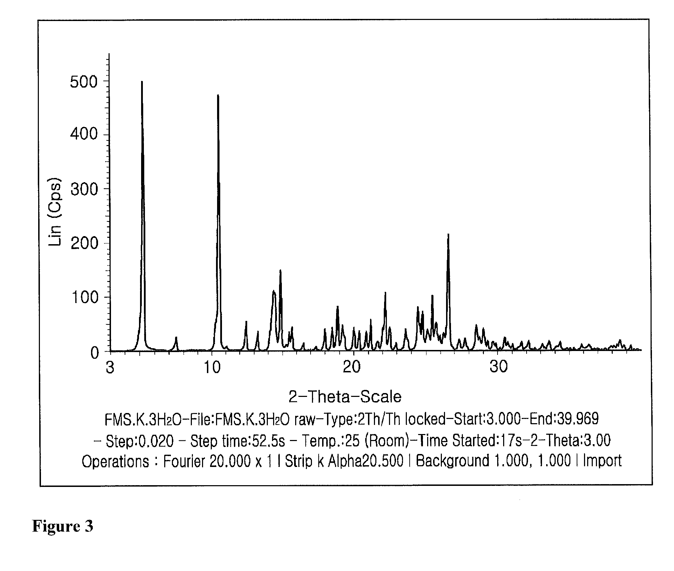

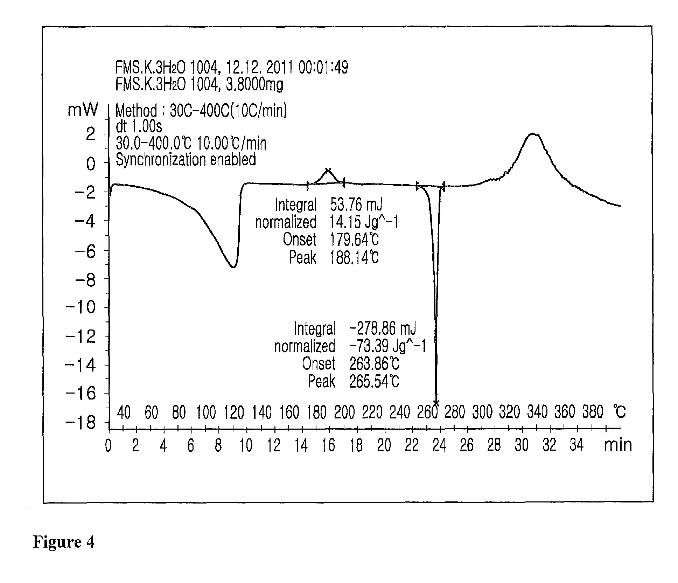

The XRD diffraction pattern of the obtained fimasartan potassium salt trihydrate by X-ray powder diffraction is shown in FIG. 3, and the DSC curve by differential scanning calorimetry is shown in FIG. 4, respectively.

Moreover, a test for loss on drying, a drying test under reduced pressure, and a test for loss on drying according to a wet granulation process were performed on the fimasartan potassium salt monohydrate and the fimasartan potassium salt trihydrate obtained in the above-described Examples to compare the two materials.

### Experimental Example 1: Test for change in water content in fimasartan potassium salt hydrates depending on reduced pressure and temperature conditions

Each 15 g of fimasartan potassium salt monohydrate and fimasartan potassium salt trihydrate was placed in a vacuum oven (OV-12) under vacuum, and the change in water content was observed at 40 °C, 60 °C, and 80 °C every 2 hours up to 8 hours using KF method.

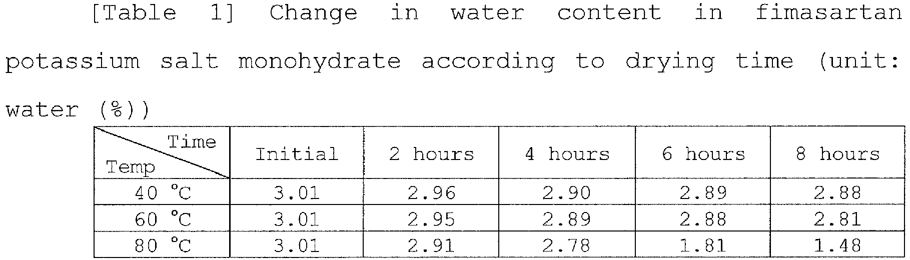

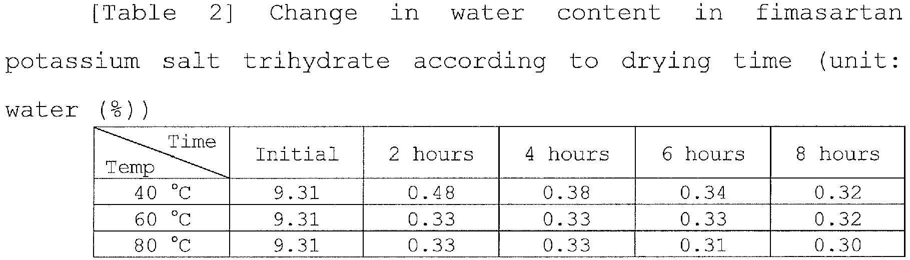

As shown in Tables 1 and 2, it could be seen that in the case of the fimasartan potassium salt trihydrate, the water content was reduced by about 95% after 2 hours. That is, it could be seen that when the fimasartan potassium salt trihydrate was dried under reduced pressure, most water was reduced within 2 hours, and thus the crystal of the trihydrate was not stably maintained. However, it could be observed that in the case of the fimasartan potassium salt monohydrate of the present invention, the change in water content with the lapse of time was not significant, and when the temperatures were 40 °C and 60 °C, the water content was reduced by less than 10% even after 8 hours. This indicates that the monohydrate has significantly increased stability under heating conditions in the drying process, coating process, etc. that may be included in a general process of preparing solid dosage forms for internal use. Moreover, it could be found from this experiment that the fimasartan potassium salt monohydrate is more advantageous in maintaining the water content with respect to the temperature, compared to the fimasartan potassium salt trihydrate.

### Experimental Example 2: Test for loss on drying in fimasartan potassium salt hydrates depending on temperature conditions

Each 5 g of fimasartan potassium salt monohydrate and fimasartan potassium salt trihydrate was placed in a moisture analyzer (Precisa XM60, Switzerland) and heated to 50 °C and 60 °C, respectively, and the loss on drying (%) was measured for 120 minutes.

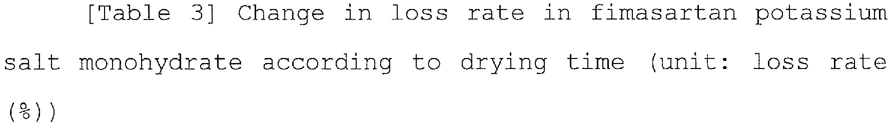

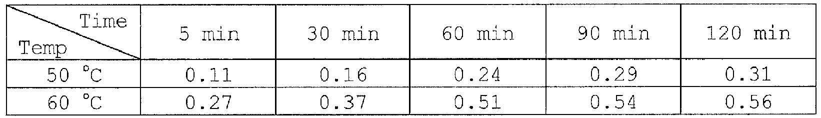

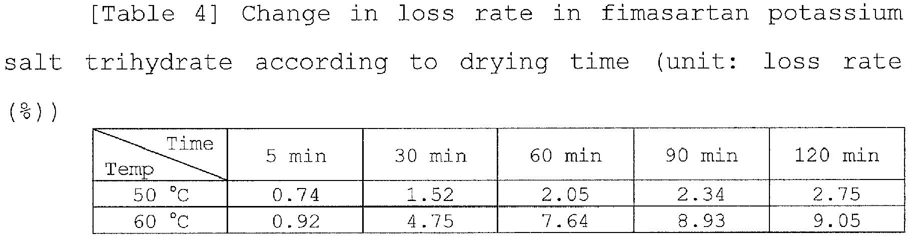

As shown in Tables 3 and 4 in the case of the fimasartan potassium salt trihydrate, 2.75% of water was reduced even at 50 °C after 2 hours and about 9% of water was reduced at 60 °C. However, in the case of the fimasartan potassium salt monohydrate of the present invention, less than 1% of water was reduced both at 50 °C and 60 °C. This indicates that the monohydrate has significantly increased stability under heating conditions in the drying process, coating process, etc. that may be included in a general process of preparing solid dosage forms for internal use. Moreover, it could be found from this experiment that the fimasartan potassium salt monohydrate is more advantageous in maintaining the water content with respect to the temperature, compared to the fimasartan potassium salt trihydrate.

### Experimental Example 3: Comparison of fimasartan potassium salt monohydrate and trihydrate in wet granulation process

In order to manufacture tablet using raw materials, a process of preparing granules (granulation process) is required for the purpose of improving the fluidity and tableting characteristics. The most widely used granulation method is wet granulation which proceeds in the order of a mixing step, a granulation step using a binder solution, and a drying step. The moisture stability of fimasartan potassium salt monohydrate was compared with that of fimasartan potassium salt trihydrate during the preparation processes.

In the wet granulation process using the fimasartan potassium salt monohydrate and trihydrate, each 3 g of the mixtures in the initial mixing step and the final drying step was placed in a moisture analyzer (Precisa XM60, Switzerland) at 105 °C, and the loss on drying (%) was measured for 20 minutes.

### Experimental Example 3-1: Wet granulation process using fimasartan potassium salt monohydrate

The following raw materials were placed in a high speed mixer (SM-5C, SEJONG) and mixed in an agitator at 100 rpm and a chopper at 2,000 rpm for 5 minutes to prepare a mixture.

| | |
|---|---|
| Fimasartan potassium salt monohydrate: | 434 g |
| Lactose hydrate: | 307.93 g |
| Microcrystalline cellulose: | 82.25 g |
| Croscarmellose sodium: | 78.8 g |

The loss on drying measured after the mixing process was 1.1%.

Apart from the above, 24.5 g of hydroxypropyl cellulose as a binder was dissolved in 165 g of purified water to prepare a binder solution. The binder solution was slowly added to the prepared mixture in a high speed mixer (SM-5C, SEJONG) and granulated in an agitator at 100 rpm and a chopper at 2,000 rpm for 3 minutes. The resulting granules were placed in a tray dryer and dried at 60 °C for 4 hours.

The loss on drying measured after the granulation and drying process was 0.9%.

### Experimental Example 3-2: Wet granulation process using fimasartan potassium salt trihydrate

The following raw materials were placed in a high speed mixer (SM-5C, SEJONG) and mixed in an agitator at 100 rpm and a chopper at 2,000 rpm for 5 minutes to prepare a mixture

| | |
|---|---|
| Fimasartan potassium salt trihydrate: | 462.07 g |
| Lactose hydrate: | 307.93 g |
| Microcrystalline cellulose: | 82.25 g |
| Croscarmellose sodium: | 78.8 g |

The loss on drying measured after the mixing process was 5.7%.

Apart from the above, 24.5 g of hydroxypropyl cellulose as a binder was dissolved in 165 g of purified water to prepare a binder solution. The binder solution was slowly added to the prepared mixture in a high speed mixer (SM-5C, SEJONG) and granulated in an agitator at 100 rpm and a chopper at 2,000 rpm for 3 minutes. The resulting granules were placed in a tray dryer and dried at 60 °C for 4 hours.

The loss on drying measured after the granulation and drying process was 1.8%.

**[Table 5] Test for change in water content in fimasartan potassium salt hydrates in the preparation of the formulation**

| Classification | Loss on drying after mixing | Loss on drying after granulation and drying | Change in water content |
|---|---|---|---|
| Fimasartan potassium salt monohydrate | 1.1% | 0.9% | -0.2% |
| Fimasartan potassium salt trihydrate | 5.7% | 1.8% | -3.9% |

As shown in Table 5, in the case of the fimasartan potassium salt trihydrate, 3.9% of change in water content occurred for 4 hours from the mixing process to the completion of the granulation process. However, it could be observed that in the case of the fimasartan potassium salt monohydrate of the present invention, only 0.2% of change in water content occurred for 4 hours from the mixing process to the completion of the granulation process. This indicates an increased stability due to the preservation of water content, etc. in the fimasartan potassium salt monohydrate.

One of the methods to prevent the dehydration of the trihydrate during the drying process is to check the loss on drying frequently and adjust the drying time to achieve an appropriate loss on drying, and the other method is to dry the trihydrate at low temperature for a long time, but both methods are inefficient in the production and may cause an increase in production cost. Therefore, it could be confirmed from this experiment that the fimasartan potassium salt monohydrate is more economical for mass production using the wet granulation process, compared to the fimasartan potassium salt trihydrate.

### Experimental Example 4: Particle size of the prepared fimasartan potassium salt monohydrate

A particle size of the fimasartan potassium salt monohydrate prepared in Example 1 was measured using HELOS System (Sympatec) with an R5 lens. Measurement of the particle size distribution was carried out using a RODOS/M Dry Powder Dispersion System and a Sympatec HELOS System.

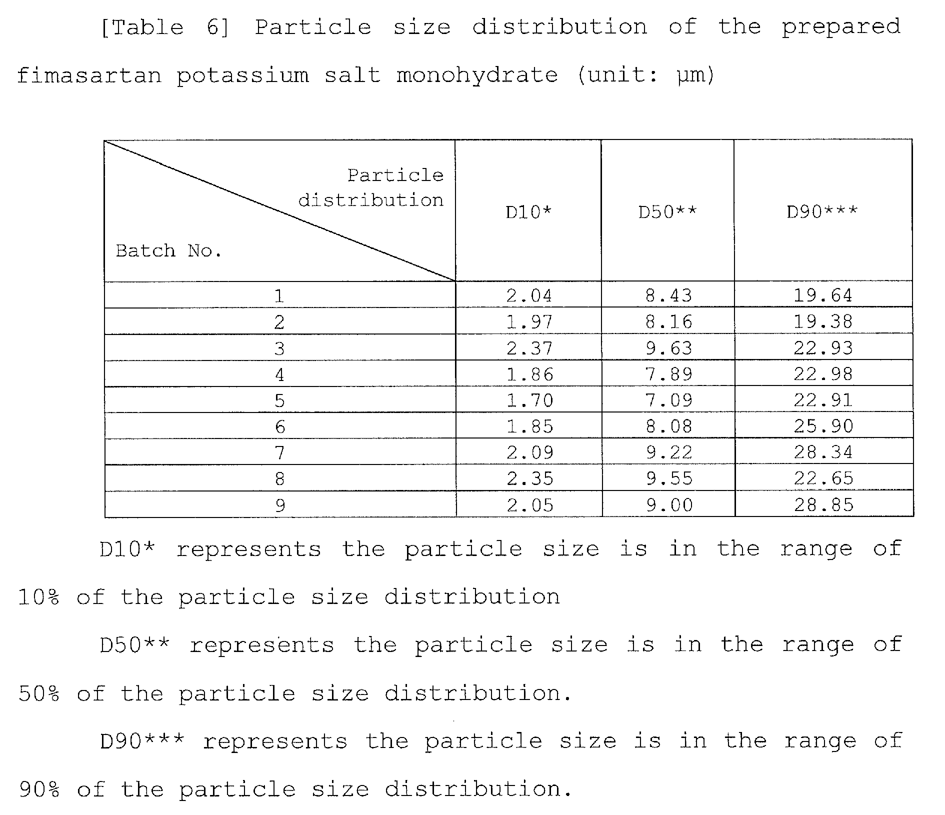

Regardless of the batch, the fimasartan potassium salt monohydrate exhibits a relatively uniform particle size, which is in the range of several µm that can be obtained by milling.

Poorly soluble drugs are required to be subjected to an essential operation process to have constant absorption and dissolution rates. However, in the case of the fimasartan potassium salt monohydrate having the uniform particle size, it is possible to effectively obtain the particles that is small and uniform, without having to be subjected to the essential operation process.

### [Industrial Applicability]

The novel fimasartan potassium salt monohydrate of the present invention is excellent in moisture and temperature stability, which makes it possible to maintain its stable state for a long time without any change in water content, and has a particle size that is uniform and easy to prepare a formulation. Therefore, the novel fimasartan potassium salt monohydrate of the present invention can maintain its stable state during the preparation process of the formulation without any change in water content, which is easy to prepare the formulation and effective for mass production.

## Claims

1. A fimasartan potassium salt monohydrate.

2. A crystalline form of fimasartan potassium salt monohydrate of claim 1, wherein the crystalline form of fimasartan potassium salt monohydrate has X-ray powder diffraction spectrum peaks using Cu-Kα radiation in diffraction angles 2θ value shown at 6.67±0.2, 7.62±0.2, 11.03+0.2, 15.32±0.2, 16.49±0.2, 20.12±0.2, 25.65±0.2, and 27.28±0.2.

3. The crystalline form of fimasartan potassium salt monohydrate of claim 2, wherein the crystalline form of fimasartan potassium salt monohydrate has X-ray powder diffraction spectrum peaks shown in FIG. 1.

4. The crystalline form of fimasartan potassium salt monohydrate of claim 2, wherein the crystalline form of fimasartan potassium salt monohydrate has an endothermic transition temperature in differential scanning calorimetry (DSC) at about 267 °C to 268 °C when the heating rate is 10 °C/min.

5. A method for preparing a fimasartan potassium salt monohydrate, the method comprising:
crystallizing fimasartan using an organic solvent comprising at least one selected from the group consisting of methanol, ethanol, isopropanol, ethyl acetate, tetrahydrofuran, acetonitrile, acetone, and dioxane.

6. The method of claim 5, wherein the crystallizing comprises:
preparing a suspension by adding the fimasartan to the organic solvent;
preparing a reactant by adding a mixed solution comprising potassium hydroxide and 2-ethylhexanoic acid to the suspension;
obtaining a crystal by cooling the reactant; and
drying the crystal.

7. The method of claim 6, wherein the preparing the reactant is performed by adding the mixed solution to the suspension, followed by heating and stirring under reflux.

8. The method of claim 7, wherein the heating is performed at 70 °C to 90 °C.

9. A method for preparing a fimasartan potassium salt monohydrate, the method comprising:
crystallizing a fimasartan potassium salt trihydrate using an organic solvent comprising at least one selected from the group consisting of methanol, ethanol, isopropanol, ethyl acetate, tetrahydrofuran, acetonitrile, acetone, and dioxane.

10. The method of claim 9, wherein the crystallizing comprises:
preparing a reactant by adding the fimasartan potassium salt trihydrate to the organic solvent, followed by heating and stirring;
obtaining a crystal by cooling the reactant; and
drying the crystal under reduced pressure.

11. The method of claim 10, wherein the heating is performed at 70 °C to 80 °C.

12. The method of claim 9, wherein the crystallizing comprises:
preparing a reactant by adding the fimasartan potassium salt trihydrate to the organic solvent, followed by reflux;
obtaining a crystal by cooling and stirring the reactant; and
drying the crystal under reduced pressure.

13. The method of claim 12, wherein the reflux is performed for 10 minutes to 1 hour.

14. The method of claim 10 or 12, wherein the stirring is performed for 10 hours to 20 hours.

15. The method of any one of claims 6, 10 and 12, wherein the cooling is performed at 20 °C to 30 °C.

16. The method of any one of claims 6, 10 and 12, wherein the drying is performed at 35 °C to 45 °C.

17. The method of any one of claims 6, 10 and 12, wherein the drying is performed at 20 mmHg to 5 mmHg.

18. A fimasartan potassium salt monohydrate prepared by the method of any one of claims 5 to 17.

19. A formulation comprising a fimasartan potassium salt monohydrate.

20. The method of claim 6, wherein the organic solvent is isopropyl alcohol.
